# FASCICULE DE BREVET EUROPEEN

(11) **EP 2 320 808 B1**
(45) Date de publication et mention de la délivrance du brevet: **05.08.2015**
(21) Numéro de dépôt: 09761884.7
(22) Date de dépôt: 12.06.2009
(51) Int. Cl.: A61B 17/17, A61F 2/46, A61B 17/58

(54) **DISPOSITIF PERMETTANT D'ÉTABLIR UN REPÈRE ANATOMIQUE D'UN DISQUE INTERVERTÉBRAL**
VORRICHTUNG FÜR ANATOMISCHE ORTUNG EINER BANDSCHEIBE
DEVICE FOR ESTABLISHING AN ANATOMICAL REFERENCE POINT OF AN INTERVERTEBRAL DISC

(30) Priorité: 13.06.2008 FR 0803314
(43) Date de publication de la demande: 18.05.2011
(73) Titulaire: Spinevision SA, 92184 Anthony Cedex (FR)
(72) Inventeur: PETIT, Dominique, F-62180 Verton (FR); DROULOUT, Thomas, F-78300 Poissy (FR); AEBI, Max, CH-2503 Bienne (CH)
(74) Mandataire: Fidal Innovation
(86) Numéro de dépôt international: PCT/FR2009/000704
(87) Numéro de publication internationale: WO 2009/150333

(56) Documents cités:
- DE-U1- 29 703 947
- FR-A- 2 887 762
- US-A1- 2006 084 986
- US-A1- 2006 111 728
- US-A1- 2006 149 273
- US-A1- 2006 149 278

## Description

L'invention concerne un dispositif permettant de réaliser un repère anatomique d'un disque intervertébral en vue de son remplacement par une prothèse discale ou une cage d'ostéosynthèse par voie postérieure ou postérolatérale.

Le dispositif selon l'invention est destiné notamment, mais non exclusivement, au repérage anatomique de au moins un disque disposé entre les vertèbres lombaires L3 et la vertèbre sacrale S1.

De manière classique en soi, il est procédé au retrait d'un disque intervertébral et à la mise en place d'une prothèse discale par approche antérieure ou latérale du disque, et plus rarement par approche postérieure. Le côté de l'abord choisi pour procéder au geste chirurgical est fonction du geste à réaliser, de la préférence du chirurgien et/ou des contraintes anatomiques du patient.

Les approches par voie antérieure et latérale présentent cependant l'inconvénient d'être relativement invasive. Elle implique par ailleurs un geste chirurgical délicat d'un point de vue des risques associés et difficile d'un point de vue technique. L'étage L5-S1 est particulièrement délicat du fait qu'il requiert d'écarter l'aorte, une mauvaise appréciation du chirurgien ainsi qu'une erreur dans le geste pouvant alors entraîner des complications vasculaires. D'autres complications peuvent en outre intervenir comme des douleurs post-opératoires pouvant nécessiter le retrait de la prothèse mise en place et / ou l'ostéosynthèse totale du niveau instrumentée.

L'approche par voie postérieure présente moins de risques de complication et en outre, s'avère moins invasive. L'inconvénient de cette approche réside cependant en ce que la vision du champ opératoire est extrêmement réduite. Aussi, afin que le chirurgien puisse accéder visuellement au champ opératoire, il est alors souvent nécessaire de procéder à des retraits de matières osseuses.

US 2006/149278 décrit un dispositif selon le préambule de la revendication 1.

L'invention vise à remédier aux problèmes d'approche d'un disque intervertébral en proposant un dispositif permettant d'établir un repère anatomique du disque à remplacer préalablement à l'implantation par approche postérieure ou postéro-latérale d'un implant tel qu'une prothèse discale ou une cage d'ostéosynthèse, et ainsi pouvoir procéder, lors de l'implantation, selon une approche du disque à remplacer peu invasive, tout en assurant une implantation précise et alignée de l'implant discal en remplacement du disque retiré.

A cet effet, et selon un premier aspect, l'invention propose un dispositif permettant d'établir un repère anatomique d'un disque intervertébral pour la fixation d'au moins un instrument destiné à l'implantation, par voie postérieure ou postéro-latérale, d'un implant tel qu'une prothèse discale ou une cage d'ostéosynthèse, en remplacement du disque intervertébral. Le dispositif comprend un premier et un deuxième moyens d'ancrage osseux destinés à être fixés chacun sur une vertèbre adjacente au disque intervertébral, un élément d'accroche sur lequel l'instrument est destiné à être fixé, l'élément d'accroche étant monté entre les premier et deuxième moyens d'ancrage osseux et des éléments de réglage de la position de l'élément d'accroche par rapport à l'emplacement du disque intervertébral. Les éléments de réglage comprennent une plateforme, d'axe longitudinal BB pourvue d'un élément de visée du disque intervertébral présentant un axe géométrique, l'élément de visée étant monté mobile sur la plateforme de façon à orienter l'axe géométrique en direction du disque intervertébral, et des moyens de connexion de la plateforme aux premier et deuxième moyens d'ancrage osseux, les moyens de connexion étant agencés pour former une liaison rotule.

Avantageusement, les éléments de réglage comportent un premier et un deuxième élément de repérage du disque intervertébral à remplacer, le premier élément constituant un repère en relation avec les apophyses épineuses des vertèbres entourant le disque intervertébral à remplacer et le deuxième élément constituant un repère indiquant la direction du disque intervertébral à remplacer, le deuxième élément étant monté mobile sur le premier élément, où la plateforme et l'élément de visée constituent respectivement les premier et deuxième éléments de repérage.

Ainsi, de par les mouvements de la plateforme par rapport aux moyens d'ancrage osseux et de l'élément de visée par rapport à la plateforme, un repère anatomique du disque intervertébral à traiter est réalisé. La plateforme constitue en effet un repère en relation avec la colonne vertébrale du patient, et plus particulièrement en relation avec les apophyses épineuses des deux vertèbres au moins entourant le disque à remplacer. L'élément de visée quant à lui constitue un repère indiquant la direction du disque intervertébral à remplacer. Les degrés de liberté des éléments de réglage les uns par rapport à l'autre permettent de positionner et d'ajuster l'élément de visée par rapport au disque intervertébral et ainsi d'assurer une implantation précise et alignée de l'implant dans l'espace intervertébral formé après retrait du disque.

Avantageusement, l'élément de visée est agencé avec la plateforme de sorte à présenter un degré de liberté en translation suivant l'axe BB.

Avantageusement, l'élément de visée est agencé avec la plateforme de sorte à présenter un degré de liberté en rotation autour d'un axe perpendiculaire à l'axe CC.

Il peut être également prévu que les éléments de réglage comportent des moyens d'ajustement de l'orientation de l'élément de visée agencés pour permettre un pivotement de l'élément de visée autour d'un axe perpendiculaire à l'axe BB. Selon une configuration particulière, les moyens d'ajustement comportent une pièce intermédiaire disposée entre l'élément de visée et la plateforme, la pièce intermédiaire présentant une face convexe sur laquelle l'élément de visée est monté mobile, la pièce intermédiaire présentant un degré de liberté en translation suivant l'axe BB.

Avantageusement, l'élément d'accroche est monté coulissant le long de l'élément de visée. Il est ainsi possible d'ajuster la position du point d'accroche en fonction de l'anatomie du patient, de l'implémentation des éléments comme par exemple la profondeur de l'insertion des moyens d'ancrage osseux dans la vertèbre, ainsi que les dimensions de l'instrument d'implantation. Il est ainsi possible de mettre en oeuvre des instruments d'implantation standard.

Afin de permettre la formation d'un « tunnel » aux fins d'un nettoyage bilatéral de l'espace intervertébral ou d'un placement bilatéral du ou des implants, l'élément d'accroche comporte deux points d'accroche pour la fixation respective d'un instrument d'implantation, les deux points d'accroche étant disposés de part et d'autre de l'élément de visée.

Il peut être également prévu de ménager sur la plateforme des moyens d'accroche additionnels pour permettre la fixation du dispositif sur un équipement de maintien apte à être fixé sur un support, comme par exemple un bras articulé lui-même fixé sur la table d'opération, et pourvu de moyens d'accroche complémentaires.

Avantageusement, le dispositif comporte une barre de liaison apte à relier les premier et deuxième moyens d'ancrage entre eux, les éléments de réglage, et avantageusement la plateforme, étant montés sur la barre de liaison.

Il peut être alors prévu, selon une configuration avantageuse, que les éléments de réglage et la barre de liaison comportent des moyens d'association réciproques, lesdits moyens étant agencés pour former une liaison rotule.

Selon un mode de réalisation particulier de l'invention, les moyens d'association réciproques comportent une articulation sphérique ménagée sur la barre de liaison coopérant avec un logement de forme complémentaire ménagé sur les éléments de réglage, et avantageusement sur la plateforme, ou inversement.

Avantageusement, le dispositif comporte des premiers et second moyens destinés à bloquer respectivement d'une part la plateforme sur les premier et second moyens d'ancrage osseux et d'autre part l'élément de visée sur la plateforme.

L'invention concerne également un système permettant l'implantation d'une prothèse discale ou cage d'ostéosynthèse par voie postérieure ou postéro-latérale, le système comprenant un dispositif permettant d'établir un repère anatomique d'un disque intervertébral tel que décrit précédemment et au moins un instrument d'implantation d'une prothèse discale ou cage d'ostéosynthèse fixé sur le dispositif.

Selon une configuration particulière, l'instrument d'implantation comprend un bras de liaison dont l'une des extrémités est montée articulée sur l'élément d'accroche du dispositif, l'autre extrémité du bras étant pourvue d'une portion tubulaire courbée en direction du disque intervertébral à remplacer.

Selon une autre configuration particulière, l'instrument d'implantation comprend un bras de liaison monté fixe sur l'élément d'accroche. Ce bras de liaison est pourvu d'un moyen de réception destiné à recevoir une portion tubulaire de forme courbée ou droite, , et configuré pour maintenir la portion tubulaire dans une position orientée vers le disque intervertébral à remplacer.

D'autres objets et avantages de l'invention apparaîtront au cours de la description qui suit, faite en référence aux dessins annexés, dans lesquels :
- la figure 1 représente une vue schématique d'un dispositif permettant d'établir un repère anatomique d'un disque intervertébral à remplacer selon l'invention, le dispositif étant fixé sur une vertèbre sus-jacente ;
- la figure 2 représente une vue du dispositif de la figure 1 pourvu d'un instrument d'implantation d'une prothèse discale ou d'une cage d'ostéosynthèse ;
- la figure 3 représente une vue du dispositif de la figure 2, dans laquelle l'instrument d'implantation est illustré en position d'implantation d'une prothèse discale ou d'une cage d'ostéosynthèse ;

- la figure 4 représente une vue du dispositif de la figure 2 pourvu de deux instruments d'implantation de prothèses discales ou cages d'ostéosynthèse ;
- les figures 5a à 5d représentent les étapes de mise en place du dispositif de la figure 1 ;
- la figure 6 représente le dispositif de la figure 1 pourvu d'un instrument d'implantation, le dispositif étant fixé sur les vertèbres sus et sous-jacentes ; et
- la figure 7 représente le dispositif de la figure 6 pourvu de deux instruments d'implantation.

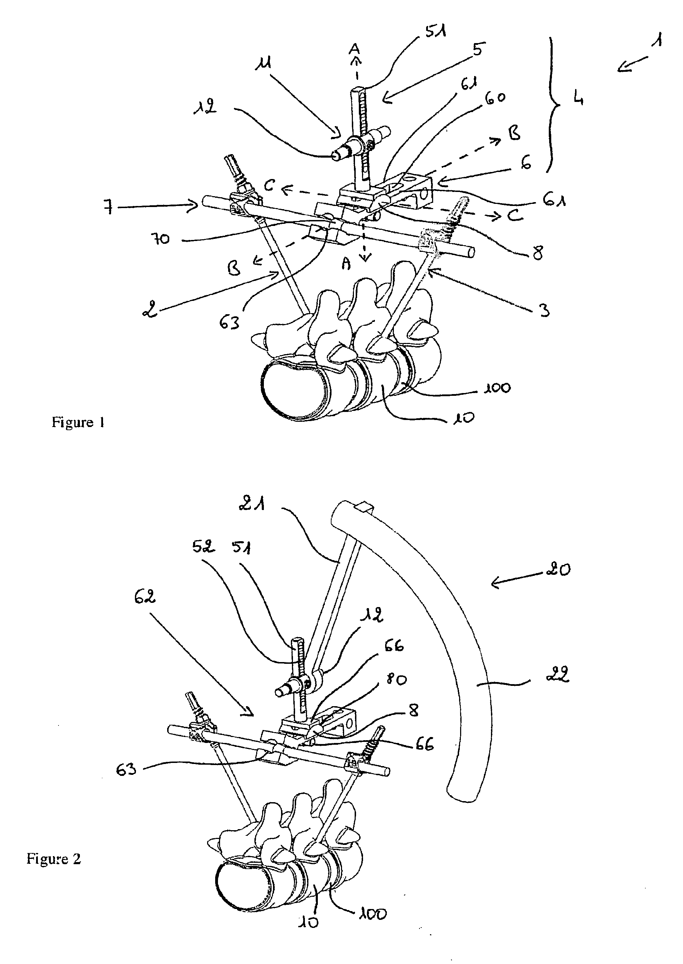

En relation avec la figure 1, il est décrit un dispositif 1 permettant d'établir un repère anatomique d'un disque intervertébral 100 à remplacer de sorte à permettre une implantation par voie postérieure ou postéro-latérale d'une prothèse discale ou d'une cage d'ostéosynthèse à l'aide d'un instrument d'implantation adapté.

Afin de faciliter la lecture de ce qui suit, le dispositif 1 permettant d'établir un repère anatomique du disque à remplacer sera par la suite appelé « dispositif de repérage 1 ».

Le dispositif de repérage 1 selon l'invention comprend deux moyens d'ancrage osseux 2, 3 et des éléments de réglage 4 montés articulés au niveau des extrémités proximales des moyens d'ancrage osseux 2, 3.

Les éléments de réglage 4 comprennent une plateforme 6 pourvue d'un élément de visée 5 du disque intervertébra! 100 à remplacer mobile ainsi que des moyens de connexion de la plateforme aux premier et deuxième moyens d'ancrage osseux 2, 3, les moyens de connexion formant une liaison rotule. Comme on le verra plus loin, la plateforme 6 a pour fonction de disposer l'élément de visée 5 dans l'alignement de l'axe vertébral du patient, et l'élément de visée d'établir la direction du disque intervertébral 100 à remplacer.

La plateforme 6 se présente sous la forme d'une barre de section rectangulaire, d'axe longitudinal BB, comportant intérieurement une fente longitudinale 60 délimitée par deux rails latéraux 61. Avantageusement, la fente 60 s'ouvre sur les faces supérieure et intérieure de la barre.

Dans le mode de réalisation décrit, la plateforme 6 est fixée sur les moyens d'ancrage osseux 2, 3 via une barre de liaison 7 disposée entre les moyens d'ancrage osseux 2, 3. Comme on le verra plus loin, la barre de liaison 7 devra être disposée horizontalement entre les moyens d'ancrages osseux 2, 3.

Avantageusement, la plateforme 6 et la barre de liaison 7 comportent des moyens d'association réciproques agencés pour former une liaison rotule. Plus particulièrement, la barre de liaison 7 comporte une articulation sphérique 70 coopérant avec un logement de forme complémentaire ménagé au niveau de l'une des extrémités 62 de la plateforme 6. Dans ce mode de réalisation, les moyens d'association réciproques constituent les moyens de connexion de la plateforme 6 aux premier et deuxième moyens d'ancrage 2, 3.

Il peut être avantageux de prévoir également une connexion articulée la barre de liaison 7 aux moyens d'ancrage osseux 2, 3. Avantageusement, cette connexion est réalisée avec un système trois dimensions, tel que le connecteur décrit dans la demande de brevet EP 1233710.

Une fois le réglage de la position de la plateforme 6 effectué, celle-ci est bloquée à l'aide d'un écrou ou vis de blocage inséré dans un logement 63 traversant, ménagé à l'extrémité 62 de la plateforme 6 et débouchant dans le logement recevant l'articulation sphérique 70 de la barre de liaison 7. Afin d'optimiser la stabilité de la plateforme, celle-ci peut également être fixée à une table opératoire par l'intermédiaire d'un bras articulé (non représenté). Pour ce faire, la plateforme comporte avantageusement des moyens d'accroche configurés pour coopérer avec des moyens d'accroche complémentaires ménagés sur le bras articulé.

L'élément de visée 5 se présente sous la forme d'une tige 51 disposée dans un plan sensiblement vertical par rapport à celui de la plateforme 6. La tige 51, présentant un axe géométrique AA, est montée mobile sur la plateforme 6 de sorte à permettre l'orientation de l'élément de visée 5, selon son axe géométrique, en direction du disque intervertébral 100 à remplacer.

Plus particulièrement, l'élément de visée 5 est agencé avec la plateforme 6 pour présenter un degré de liberté en translation suivant l'axe BB de la plateforme 6. Dans le mode de réalisation décrit, l'élément de visé 5 est monté coulissant le long des rails latéraux 61 de la plateforme 6.

Avantageusement, l'élément de visée est agencé avec la plateforme 6 pour présenter également un degré de liberté en rotation autour d'un axe CC perpendiculaire à l'axe BB de la plateforme 6. Dans le mode de réalisation décrit, le degré de liberté en rotation est obtenu à l'aide d'une pièce intermédiaire 8 disposée entre l'élément de visée 5 et la plateforme 6.

Plus particulièrement, la pièce intermédiaire 8 présente une face convexe 80 sur laquelle l'élément de visée 5 est monté mobile, la face convexe étant orientée de sorte à permettre un pivotement de l'élément de visée 5 en direction de l'une ou l'autre des extrémités de la plateforme d'alignement 6 (pivotement autour de l'axe CC). La pièce intermédiaire 8 est montée coulissante le long des rails latéraux 61 de la plateforme 6.

La pièce intermédiaire 8 ainsi décrite constitue des moyens d'ajustement de l'orientation de l'élément de visée 5 par rapport au disque intervertébral 100 à remplacer. Il s'agit bien entendu d'un exemple de réalisation, l'invention n'étant pas limitée à de tels moyens d'ajustement.

Une fois la position de l'élément de visée 5 sur la plateforme 6 réglée et son orientation par rapport au disque intervertébral 100 ajustée, celui-ci est bloqué en position à l'aide de moyens 66 formant étau de serrage.

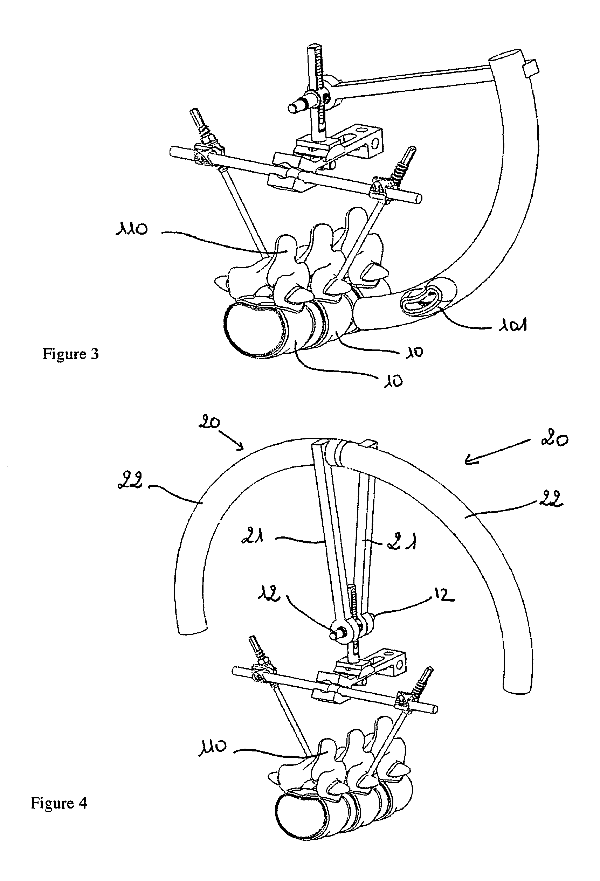

Afin de permettre la fixation d'un instrument d'implantation d'une prothèse discale sur le dispositif de repérage 1, les éléments de réglage 4 comportent un élément d'accroche 11 pourvu d'au moins un point d'accroche 12 se présentant sous la forme d'un bras s'étendant parallèlement à l'axe de la plateforme 6. Dans le mode de réalisation décrit, l'élément d'accroche 11 présente deux points d'accroche 12 disposés opposés l'un par rapport à l'autre. Ainsi, le dispositif de repérage 1 pourra être équipé de deux instruments d'implantation comme illustré sur la figure 4.

Afin de permettre la mise en oeuvre un instrument d'implantation standard, l'élément d'accroche 11 est avantageusement monté coulissant le long de la tige 51 de l'élément de visée 5. De la sorte, il est possible de régler la hauteur du point d'accroche de l'instrument d'implantation par rapport au disque intervertébral à remplacer et ainsi d'ajuster la position du point d'accroche en fonction de l'anatomie du patient, de l'implémentation des éléments constituant le dispositif de repérage 1 (comme par exemple la profondeur de l'insertion des moyens d'ancrage osseux dans la vertèbre) ainsi que les dimensions de l'instrument d'implantation.

Le blocage de l'élément d'accroche 11 sur la tige 51 est obtenu par vissage de celui-ci sur la tige 51 de l'élément de visée 5. A cet effet, il sera avantageux de prévoir sur la tige 51 de l'élément de visée 5 un crantage 52 coopérant avec un crantage complémentaire de l'élément d'accroche 11.

L'instrument d'implantation 20 associé au dispositif de repérage 1 précédemment décrit comporte un bras de liaison 21 apte à être monté articulé sur le point d'accroche 12 de l'élément d'accroche 11 au niveau de l'une de ses extrémités, l'autre extrémité étant pourvue d'une portion tubulaire 22 courbée, selon un rayon déterminé, en direction du disque intervertébral 100 à remplacer (figure 2). Comme on le verra plus loin, le positionnement du point d'accroche 12 établi après réglage et ajustement des éléments 4, à savoir la barre de liaison 7, la plateforme 6 et l'élément de visée 5, par rapport au disque intervertébral 100 à remplacer, permet que l'instrument d'implantation, lorsqu'il est déplacé dans sa position d'implémentation, se positionne automatiquement et précisément dans l'alignement du plan du disque intervertébral 100 à remplacer, permettant ainsi de procéder au retrait du disque intervertébral, au nettoyage de l'espace intervertébral et à la mise en place de la prothèse discale 101 ou d'une cage d'ostéosynthèse (figure 3).

Il est à noter que la portion courbée est agencée sur le bras de liaison 21 de sorte que lorsque le dispositif de repérage 1 est équipé de deux instruments d'implantation, les portions courbées 22 de chacun des instruments d'implantation sont disposés dans un même plan (figure 3).

Le procédé pour repérer anatomiquement le disque intervertébral 100 à remplacer et établir la voie d'approche au moyen du dispositif de repérage 1 précédemment décrit est le suivant.

La première étape consiste à ancrer les deux moyens d'ancrage osseux 2, 3 dans la vertèbre 10. Dans le mode de réalisation décrit, les moyens d'ancrage osseux 2, 3 sont ancrés sur la même vertèbre. Il pourra s'agir indépendamment de la vertèbre sus ou sous jacente du disque intervertébral 100 à remplacer. La barre de liaison 7 est ensuite fixée entre les deux moyens d'ancrage osseux 2, 3.

Les étapes qui suivent sont destinées à permettre un parfait centrage de la prothèse dans l'espace intervertébral formé une fois le disque intervertébral retiré lors de la mise en oeuvre de l'instrument d'implantation associé.

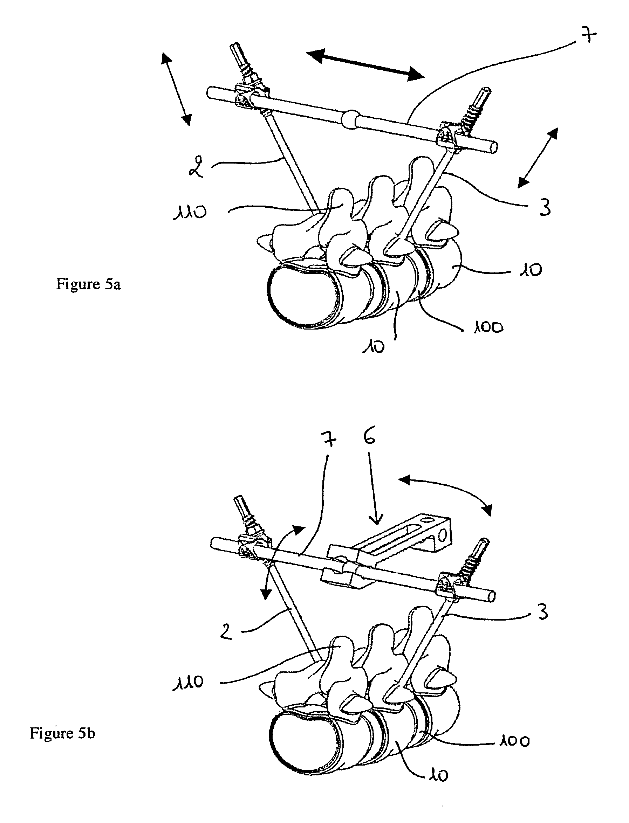

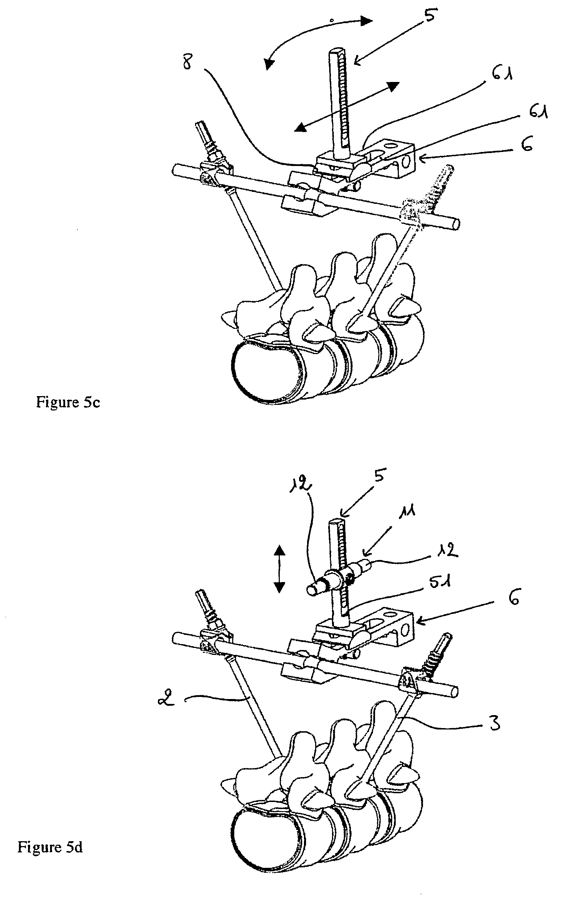

Il est procédé dans un premier temps à la mise en place de la barre de liaison 7 entre les deux moyens d'ancrage osseux 2, 3. La barre de liaison 7 devra être positionnée dans le plan horizontal et l'articulation 70 formant rotule de la barre de liaison 7 devra être disposée dans l'alignement des apophyses épineuses 110 des vertèbres 10, comme représenté sur la figure 5a par les deux séries de flèches. Le contrôle de l'horizontalité de la barre de liaison 7 sera réalisé à l'aide d'un niveau.

Une fois l'horizontalité de la barre de liaison 7 et l'alignement de l'articulation assurés, la plateforme 6, montée sur la barre de liaison 7 au niveau de l'articulation, est ajustée de manière à être positionnée dans un plan sensiblement perpendiculaire au plan du disque 100 considéré, la position de la plateforme 6 dépendant en effet de la lordose du patient (figure 5b). L'ajustement est rendu possible du fait de la présence de la liaison rotule entre la barre de liaison 7 et la plateforme 6. Le contrôle de la position de la plateforme d'alignement 6 peut être réalisé à l'aide de la ligne visible formée par les apophyses épineuses vue sur l'arrière du patient. Une fois la position de la plateforme 6 déterminée, cette dernière est alors bloquée en position à l'aide des premiers moyens de blocage.

Il est ensuite procédé à l'ajustement de l'élément de visée 5 vis-à-vis du disque intervertébral 100 en procédant au déplacement en translation de l'élément de visée le long des rails latéraux 61 de la plateforme 6 et/ou au pivotement de l'élément de visée 5 sur la pièce intermédiaire 8, de sorte à orienter l'axe géométrique de l'élément de visée 5 en direction du disque intervertébral 100 à remplacer. Les mouvements de l'élément de visée 5 sont représentés sur la figure 5c par les deux flèches. Un contrôle des ajustements est effectué par la réalisation d'une fluoroscopie latérale. Une fois la position de l'élément de visée 5 souhaitée atteinte, ce dernier est alors bloqué en position à l'aide des seconds moyens de blocage.

Il est alors procédé en dernier lieu au réglage de la hauteur du point d'accroche 12 de l'instrument d'implantation sur la tige 51 de l'élément de visée 5 en faisant coulisser l'élément d'accroche 11 le long de la tige 51. Une fois la hauteur requise de l'élément d'accroche 11 atteinte, celui-ci est bloqué sur la tige. Comme précédemment, le contrôle de la position de l'élément d'accroche 11 est effectué par la réalisation d'une fluoroscopie latérale.

Le dispositif de repérage 1 ainsi calibré est prêt à recevoir l'instrumentation d'implantation 20.

Il est à noter que, afin de montrer de manière claire les différents ajustements intervenant dans la mise en place du dispositif de repérage 1, tous les éléments de réglage 4 n'ont pas été représentés sur les figures 4a à 4c. Il est bien entendu évident que les éléments de réglage 4 ne se montent pas au fur et à mesure des ajustements associés comme cela apparaît sur les figures précitées.

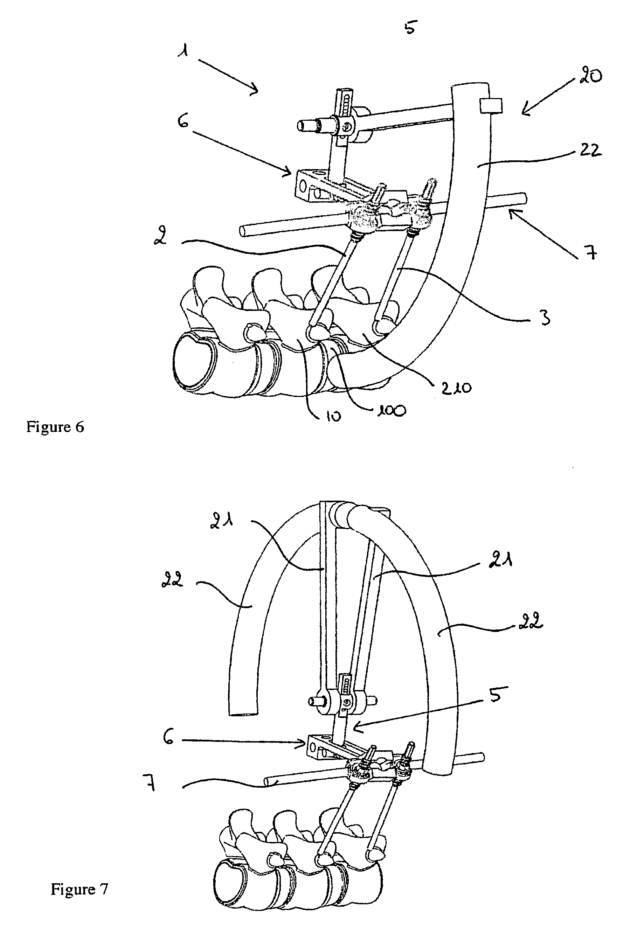

Dans l'exemple illustré, les moyens d'ancrage osseux sont fixés sur une même vertèbre. Il est bien entendu évident que le dispositif de repérage 1 pourra être ancré sur deux vertèbres distinctes 10, 210 disposées de part et d'autre du disque intervertébral 100 à remplacer, comme illustré sur les figures 6 et 7. Les vertèbres destinés à recevoir les moyens d'ancrage osseux peuvent être distantes l'une de l'autre, laissant au moins une vertèbre intermédiaire sans moyen d'ancrage.

Dans cette configuration, le dispositif de repérage 1 comprendra également une barre de liaison 7, la plateforme d'alignement 6 étant disposée perpendiculairement à l'axe des apophyses épineuses et dans l'axe du disque intervertébral. Le degré de liberté en translation de l'élément de visée 5 est alors prévu pour un positionnement de celui-ci dans l'axe des apophyses épineuses.

L'invention est décrite dans ce qui précède à titre d'exemple. Il est entendu que l'homme du métier est à même de réaliser différentes variantes de réalisation de l'invention sans pour autant sortir du cadre de l'invention, defini dans les revendications.

## Revendications

1. Dispositif (1) permettant d'établir un repère anatomique d'un disque intervertébral (100) pour la fixation d'au moins un instrument destiné à l'implantation, par voie postérieure ou postéro-latérale, d'une prothèse discale ou d'une cage d'ostéosynthèse en remplacement du disque intervertébral, le dispositif (1) comprenant :
- un premier et un deuxième moyens d'ancrage osseux (2, 3) destinés à être fixés chacun sur une vertèbre (10),
- un élément d'accroche (11) sur lequel l'instrument est destiné à être fixé, l'élément d'accroche (11) étant apte à être monté entre les premier et deuxième moyens d'ancrage osseux (2, 3),
- des éléments de réglage (4) de la position de l'élément d'accroche (11) par rapport à l'emplacement du disque intervertébral (100), lesdits éléments de réglage (4) comprenant une plateforme (6), d'axe longitudinal (BB), pourvue d'un élément de visée (5) du disque intervertébral (100) présentant un axe géométrique (AA), l'élément de visée (5) étant monté mobile sur la plateforme (6) de façon à orienter l'axe géométrique (AA), en direction du disque intervertébral (100),
**caractérisé en ce que** les éléments de réglage comprennent en outre des moyens de connexion (7) de la plateforme aux premier et deuxième moyens d'ancrage osseux (2, 3), lesdits moyens de connexion étant agencés pour former une liaison rotule (70).

2. Dispositif (1) permettant d'établir un repère anatomique d'un disque intervertébral (100) selon la revendication 1, **caractérisé en ce que** les éléments de réglage (4) comportent un premier et un deuxième élément de repérage du disque intervertébral (100) à remplacer, le premier élément constituant un repère en relation avec les apophyses épineuses des vertèbres entourant le disque intervertébral à remplacer, le deuxième élément constituant un repère indiquant la direction du disque intervertébral (100) à remplacer, le deuxième élément étant monté mobile sur le premier élément, où
la plateforme (6) et l'élément de visée (5) constituent respectivement les premier et deuxième éléments de repérage.

3. Dispositif (1) permettant d'établir un repère anatomique d'un disque intervertébral (100) selon l'une quelconque des revendications 1 à 2 **caractérisé en ce que** l'élément de visée (5) est agencé avec la plateforme (6) de sorte à présenter un degré de liberté en translation suivant l'axe longitudinal (BB).

4. Dispositif (1) permettant d'établir un repère anatomique d'un disque intervertébral (100) selon l'une quelconque des revendications 1 à 3, **caractérisé en ce que** l'élément de visée (5) est agencé avec la plateforme (6) de sorte à présenter un degré de liberté en rotation autour d'un axe perpendiculaire à l'axe longitudinal (BB).

5. Dispositif (1) permettant d'établir un repère anatomique d'un disque intervertébral (100) selon l'une quelconque des revendications 1 à 4 **caractérisé en ce que** les éléments de réglage (4) comportent des moyens d'ajustement de l'orientation de l'élément de visée (5) agencés pour permettre un pivotement de l'élément de visée (5) autour d'un axe perpendiculaire à l'axe longitudinal (BB).

6. Dispositif (1) permettant d'établir un repère anatomique d'un disque intervertébral (100) selon la revendication précédente, **caractérisé en ce que** les moyens d'ajustement comportent une pièce intermédiaire (8) disposée entre l'élément de visée (5) et la plateforme (6), la pièce intermédiaire (8) présentant une face convexe (80) sur laquelle l'élément de visée est montée mobile, la pièce intermédiaire (8) présentant un degré de liberté en translation suivant l'axe longitudinal (BB).

7. Dispositif (1) permettant d'établir un repère anatomique d'un disque intervertébral (100) selon l'une quelconque des revendications 1 à 6, **caractérisé en ce que** l'élément d'accroche (11) est monté coulissant le long de l'élément de visée (5).

8. Dispositif (1) permettant d'établir un repère anatomique d'un disque intervertébral (100) selon l'une quelconque des revendications 1 à 7 **caractérisé en ce que** l'élément d'accroche (11) comporte deux points d'accroche pour la fixation respective d'un instrument d'implantation, les deux points de fixation étant disposés de part et d'autre de l'élément de visée (5).

9. Dispositif (1) permettant d'établir un repère anatomique d'un disque intervertébral (100) selon l'une quelconque des revendications 1 à 8 **caractérisé en ce que** le dispositif comporte des premiers et second moyens destinés à bloquer respectivement la plateforme (6) sur les premier et second moyens d'ancrage osseux (2, 3) et l'élément de visée (5) sur la plateforme (6).

10. Dispositif (1) permettant d'établir un repère anatomique d'un disque intervertébral (100) selon l'une quelconque des revendications 1 à 9, **caractérisé en ce que** la plateforme (6) comporte en outre des moyens d'accroche additionnels permettant la fixation du dispositif (1) sur un équipement de maintien apte à être fixé sur un support et pourvu de moyens d'accroche complémentaires.

11. Dispositif (1) permettant d'établir un repère anatomique d'un disque intervertébral (100) selon l'une quelconque des revendications précédentes, **caractérisé en ce que** le dispositif comporte une barre de liaison (7) apte à relier les premier et deuxième moyens d'ancrage entre eux, les éléments de réglage (4) étant montés sur la barre de liaison (7).

12. Dispositif (1) permettant d'établir un repère anatomique d'un disque intervertébral (100) selon la revendication précédente, **caractérisé en ce que** les éléments de réglage (4) et la barre de liaison (7) comportent des moyens d'association réciproques agencés pour former une liaison rotule.

13. Dispositif (1) permettant d'établir un repère anatomique d'un disque intervertébral (100) selon la revendication précédente, **caractérisé en ce que** les moyens d'association réciproques comportent une articulation sphérique ménagée sur l'un des éléments constitués par les éléments de réglage (4) et la barre de liaison (7), l'articulation sphérique coopérant avec un logement de forme complémentaire ménagé sur l'autre élément.

14. Système permettant l'implantation d'une prothèse discale ou cage d'ostéosynthèse par voie postérieure ou postéro-latérale, le système comprenant le dispositif (1) permettant d'établir un repère anatomique d'un disque intervertébral (100) selon l'une quelconque des revendications précédentes et au moins un instrument d'implantation (20) d'une prothèse discale ou cage d'ostéosynthèse fixé sur le dispositif (1).

15. Système permettant l'implantation d'une prothèse discale ou cage d'ostéosynthèse selon la revendication 14, **caractérisé en ce que** l'instrument d'implantation comprend un bras de liaison (21) dont l'une des extrémités est montée articulée sur l'élément d'accroche (11) du dispositif (1), l'autre extrémité du bras étant pourvue d'une portion tubulaire courbée (22) en direction du disque intervertébral à remplacer.

16. Système permettant l'implantation d'une prothèse discale ou cage d'ostéosynthèse selon la revendication 14, **caractérisé en ce que** l'instrument d'implantation comprend un bras de liaison monté fixe sur l'élément d'accroche (11), le bras de liaison étant pourvu d'un moyen de réception destiné à recevoir une portion tubulaire de forme courbée ou droite, et configuré pour maintenir la portion tubulaire dans une position orientée vers le disque intervertébral à remplacer.

## Patentansprüche

1. Vorrichtung (1), die die Festlegung eines anatomischen Bezugspunktes einer Bandscheibe (100) für die Fixierung wenigstens eines Instruments zulässt, das zur Einsetzung auf hinterem oder hinterem / lateralem Wege einer Wirbelprothese oder eines Osteosynthesekäfigs als Ersatz für die Bandscheibe bestimmt ist, wobei die Vorrichtung (1) umfasst:
- ein erstes und ein zweites Verankerungsmittel (2, 3) im Knochen, die jeweils zur Befestigung auf einem Wirbel (10) bestimmt sind,
- Befestigungselement (11), auf dem das Instrument zur Befestigung bestimmt ist, wobei das Befestigungselement (11) geeignet ist, zwischen den ersten und den zweiten Verankerungsmitteln im Knochen (2, 3) montiert zu werden,
- Einstellelemente (4) der Position des Befestigungselements (11) im Verhältnis zur Stelle der Bandscheibe (100), wobei die genannten Einstellelemente (4) eine Plattform (6) mit Längsachse (BB) umfassen, die mit einem Peilelement (5) der eine geometrische Achse (AA) aufweisenden Bandscheibe (100) versehen ist, wobei das Peilelement (5) derart mobil auf der Plattform (6) montiert ist, dass die geometrische Achse (AA) in Richtung der Bandscheibe (100) ausgerichtet ist,
**dadurch gekennzeichnet, dass** die Einstellelemente darüber hinaus Anschlussmittel (7) der Plattform an die ersten und zweiten Verankerungsmittel (2, 3) im Knochen umfassen, wobei die genannten Anschlussmittel angeordnet sind, um eine Kugelverbindung (70) zu formen.

2. Vorrichtung (1), die die Herstellung eines anatomischen Bezugspunktes einer Bandscheibe (100) gemäß Anspruch 1 zulässt, **dadurch gekennzeichnet, dass** die Einstellelemente (4) ein erstes und ein zweites Markierungselement der zu ersetzenden Bandscheibe (100) umfassen, wobei das erste Element einen Bezugspunkt in Bezug auf die Dornfortsätze der die zu ersetzende Bandscheibe umgebenden Wirbel darstellt, wobei das zweite Element einen die Richtung der zu ersetzenden Bandscheibe (100) bildende Markierung angibt, wobei das zweite Element mobil auf dem ersten Element ist, wobei die Plattform (6) und das Peilelement (5) jeweils die ersten und zweiten Markierungselemente darstellen.

3. Vorrichtung (1), die die Herstellung einer anatomischen Markierung einer Bandscheibe (100) gemäß Anspruch 1 bis 2 zulässt, **dadurch gekennzeichnet, dass** das Peilelement (5) mit der Plattform (6) derart angeordnet ist, dass es einen Freiheitsgrad in Translation gemäß der Längsachse (BB) aufweist.

4. Vorrichtung (1), die die Herstellung einer anatomischen Markierung einer Bandscheibe (100) gemäß Anspruch 1 bis 3 zulässt, **dadurch gekennzeichnet, dass** das Peilelement (5) mit der Plattform (6) derart angeordnet ist, dass es einen Freiheitsgrad in Rotation um eine zur Achse (BB) lotrechte Achse aufweist.

5. Vorrichtung (1), die die Herstellung einer anatomischen Markierung einer Bandscheibe (100) gemäß Anspruch 1 bis 4 zulässt, **dadurch gekennzeichnet, dass** die Einstellelemente (4) Anpassmittel der Ausrichtung des Peilelements (5) umfassen, die angeordnet sind, um ein Schwenken des Peilelements (5) um eine zur Längsachse (BB) lotrechte Achse zu erlauben.

6. Vorrichtung (1), die die Herstellung einer anatomischen Markierung einer Bandscheibe (100) gemäß dem voranstehenden Anspruch zulässt, **dadurch gekennzeichnet, dass** die Anpassmittel ein zwischen dem Peilmittel (5) und der Plattform (6) angeordnetes Zwischenstück (8) umfassen, wobei das Zwischenstück (8) eine konvexe Seite (80) aufweist, auf der das Peilelement mobil montiert ist, wobei das Zwischenstück (8) einen Freiheitsgrad in Translation gemäß der Längsachse (BB) aufweist.

7. Vorrichtung (1), die die Herstellung einer anatomischen Markierung einer Bandscheibe (100) gemäß Anspruch 1 bis 6 zulässt, **dadurch gekennzeichnet, dass** das Befestigungselement (1) gleitend entlang des Peilelements (5) montiert ist.

8. Vorrichtung (1), die die Herstellung einer anatomischen Markierung einer Bandscheibe (100) gemäß Anspruch 1 bis 7 zulässt, **dadurch gekennzeichnet, dass** das Befestigungselement (1) zwei Befestigungspunkte für die jeweilige Fixierung eines Implantationsinstruments umfasst, wobei die zwei Fixierungspunkte auf jeder Seite des Peilelements (5) angeordnet sind.

9. Vorrichtung (1), die die Herstellung einer anatomischen Markierung einer Bandscheibe (100) gemäß Anspruch 1 bis 8 zulässt, **dadurch gekennzeichnet, dass** die Vorrichtung erste und zweite Mittel umfasst, die dazu bestimmt sind, die Plattform (6) jeweils auf dem ersten und zweiten Verankerungsmittel (2, 3) im Knochen und das Peilelement (5) auf der Plattform (6) zu blockieren.

10. Vorrichtung (1), die die Herstellung einer anatomischen Markierung einer Bandscheibe (100) gemäß 1 bis 9 zulässt, **dadurch gekennzeichnet, dass** die Plattform (6) darüber hinaus zusätzliche Verankerungsmittel umfasst, die die Fixierung der Vorrichtung (1) auf einer Festhalteausrüstung zulassen, die geeignet ist, auf einem Träger fixiert zu werden und mit komplementären Verankerungsmitteln versehen ist.

11. Vorrichtung (1), die die Herstellung einer anatomischen Markierung einer Bandscheibe (100) gemäß einem der voranstehenden Ansprüche zulässt, **dadurch gekennzeichnet, dass** die Vorrichtung eine Verbindungsstange (7) umfasst, die geeignet ist, das erste und zweite Verankerungsmittel miteinander zu verbinden, wobei die Einstellmittel (4) auf der Verbindungsstange (7) montiert sind.

12. Vorrichtung (1), die die Herstellung einer anatomischen Markierung einer Bandscheibe (100) gemäß dem voranstehenden Anspruch zulässt, **dadurch gekennzeichnet, dass** die Einstellelemente (4) und die Verbindungsstange (7) Mittel zur gegenseitigen Zuordnung umfassen, die angeordnet sind, um eine Kugelverbindung zu formen.

13. Vorrichtung (1), die die Herstellung einer anatomischen Markierung einer Bandscheibe (100) gemäß dem voranstehenden Anspruch zulässt, **dadurch gekennzeichnet, dass** die gegenseitigen Zuordnungsmittel eine sphärische Artikulation umfassen, die auf einem der Elemente angeordnet ist, die durch die Einstellmittel (4) und die Verbindungsstange (7) gebildet sind, wobei die sphärische Artikulation mit einer Aufnahme in auf dem anderen Element angeordneten sphärischen Form zusammenwirkt.

14. System, das die Einsetzung einer Wirbelprothese oder eines Osteosynthesekäfigs auf hinterem oder hinterem / lateralem Wege zulässt, wobei das System die Vorrichtung (1) umfasst, die die Herstellung einer anatomischen Markierung einer Bandscheibe (100) gemäß einem der voranstehenden Ansprüche und wenigstens eines Einsetzungsinstruments (20) einer Wirbelprothese oder eines auf der Vorrichtung (1) fixierten Osteosynthesekäfigs erlaubt.

15. System, das die Einsetzung einer Wirbelprothese oder eines Osteosynthesekäfigs gemäß Anspruch 14 zulässt, **dadurch gekennzeichnet, dass** das Einsetzungsinstrument einen Verbindungsarm (21) umfasst, dessen eines Ende artikuliert auf dem Verankerungselement (11) der Vorrichtung (1) montiert ist, wobei das andere Ende des Arms mit einem gekrümmten, röhrenförmigen Abschnitt (22) in Richtung des zu ersetzenden Wirbels versehen ist.

16. System, das die Einsetzung einer Wirbelprothese oder eines Osteosynthesekäfigs gemäß Anspruch 4 zulässt, **dadurch gekennzeichnet, dass** das Einsetzungsinstrument einen Verbindungsarm umfasst, der fest auf dem Verankerungselement (11) montiert ist, wobei der Verbindungsarm mit einem Aufnahmemittel verstehen ist, das dazu bestimmt ist, einen geraden oder gekrümmten röhrenförmigen Abschnitt aufzunehmen, und konfiguriert ist, um den röhrenförmigen Abschnitt in einer zur zu ersetzenden Bandscheibe ausgerichteten Position zu halten.

## Claims

1. A device (1) for establishing an anatomical reference point of an intervertebral disc (100) for the attachment of at least one instrument intended for the implantation, through a posterior or posterolateral approach, of a disc prosthesis or an osteosynthesis cage as a replacement for the intervertebral disc, with the device (1) comprising:
- a first and a second means for bone anchorage (2, 3) each intended to be attached to a vertebra (10),
- a coupling element (11) whereon the instrument is intended to be attached, with the coupling element (11) being adapted to be mounted between the first and the second means for bone anchorage (2, 3),
- elements (4) for adjusting the position of the coupling element (11) relative to the location of the intervertebral disc (100), with said adjusting elements (4) comprising a platform (6), having a longitudinal axis (BB), provided with an element (5) for sighting the intervertebral disc (100) having a geometrical axis (AA), with the sighting element (5) being mounted to be movable on the platform (6) so as to orient the geometrical axis (AA) toward the intervertebral disc (100),
**characterized in that** the adjusting elements further comprise means for connecting (7) the platform to the first and second means for bone anchorage (2, 3), with said connecting means being so arranged as to form a spherical joint (70).

2. A device (1) for establishing an anatomical reference point of an intervertebral disc (100) according to claim 1, **characterized in that** the adjusting elements (4) comprise a first and a second element for spotting the intervertebral disc (100) to be replaced, with the first element forming a reference point relative to the spines of the vertebrae surrounding the intervertebral disk to be replaced, with the second element forming a reference point indicating the direction of the intervertebral disc (100) to be replaced, with the second element being mounted so as to be movable on the first element, wherein
the platform (6) and the sighting element (5) are the first and the second spotting elements, respectively.

3. A device (1) for establishing an anatomical reference point of an intervertebral disc (100) according to any one of claims 1 to 2, **characterized in that** the sighting element (5) is so arranged with the platform (6) as to have one degree of freedom in translation about an axis perpendicular to the longitudinal axis (BB).

4. A device (1) for establishing an anatomical reference point of an intervertebral disc (100) according to any one of claims 1 to 3, **characterized in that** the sighting element (5) is so arranged with the platform (6) as to have one degree of freedom in rotation about an axis perpendicular to the longitudinal axis (BB).

5. A device (1) for establishing an anatomical reference point of an intervertebral disc (100) according to any one of claims 1 to 4, **characterized in that** the adjusting elements (4) comprise means for adjusting the orientation of the sighting element (5) so arranged as to enable the sighting element (5) to pivot about an axis perpendicular to the longitudinal axis (BB).

6. A device (1) for establishing an anatomical reference point of an intervertebral disc (100) according to the preceding claim, **characterized in that** the adjusting means comprise an intermediate part (8) positioned between the sighting element (5) and the platform (6), with the intermediate part (8) having a convex face (80) whereon the sighting element is mounted to be movable, with the intermediate part (8) having one degree of freedom in translation along the longitudinal axis (BB).

7. A device (1) for establishing an anatomical reference point of an intervertebral disc (100) according to any one of claims 1 to 6, **characterized in that** the coupling element (11) is mounted to be able to slide along the sighting element (5).

8. A device (1) for establishing an anatomical reference point of an intervertebral disc (100) according to any one of claims 1 to 7, **characterized in that** the coupling element (11) comprises two coupling points for the respective attachment of an implantation instrument, with the two coupling points being arranged on either side of the sighting element (5).

9. A device (1) for establishing an anatomical reference point of an intervertebral disc (100) according to any one of claims 1 to 8, **characterized in that** the device comprises first and second means for locking the platform (6) on the first and the second means for bone anchorage (2, 3) and the sighting element (5) on the platform (6), respectively.

10. A device (1) for establishing an anatomical reference point of an intervertebral disc (100) according to any one of claims 1 to 9, **characterized in that** the platform (6) further comprises additional coupling means for fixing the device (1) on a holding device adapted to be attached on a support and provided with complementary coupling means.

11. A device (1) for establishing an anatomical reference point of an intervertebral disc (100) according to any one of the preceding claims, **characterized in that** the device comprises a tie rod (7) adapted to link the first and second means for bone anchorage together, with the adjusting elements (4) being mounted on the tie rod (7).

12. A device (1) for establishing an anatomical reference point of an intervertebral disc (100) according to the preceding claim, **characterized in that** the adjusting elements (4) and the tie rod (7) comprise mutually associating means so arranged as to form a spherical joint.

13. A device (1) for establishing an anatomical reference point of an intervertebral disc (100) according to the preceding claim, **characterized in that** the mutually associating means comprise a ball joint provided on one of the elements constituted by the adjusting elements (4) and the tie rod (7), with the ball joint cooperating with a matching recess provided on the other element.

14. A system for implanting a disc prosthesis or an osteosynthesis cage, through a posterior or posterolateral approach, with the system comprising the device (1) for establishing an anatomical reference point of an intervertebral disc (100) according to any one of the preceding claims, and at least one instrument (20) for implanting a disc prosthesis or an osteosynthesis cage attached to the device (1).

15. A system for implanting a disc prosthesis or an osteosynthesis cage according to claim 14, **characterized in that** the implantation instrument comprises a link arm (21), one end of which is so mounted as to be articulated on the coupling element (11) of the device (1), with the other end of the arm being provided with a tubular portion (22) bent towards the intervertebral disk to be replaced.

16. A system for implanting a disc prosthesis or an osteosynthesis cage according to claim 14, **characterized in that** the implantation instrument comprises a link arm mounted to be stationary on the coupling element (11), with the link arm being provided with receiving means intended for receiving a tubular portion having a curved or straight shape, and so configured as to hold the tubular portion in a position oriented toward the intervertebral disk to be replaced.
